# EUROPEAN PATENT APPLICATION

(11) **EP 2 942 067 A1**
(43) Date of publication of application: **11.11.2015**
(21) Application number: 15161482.3
(22) Date of filing: 27.03.2015
(51) Int. Cl.: A61L 15/60, A61F 13/53

(54) **COMPOSITION FOR ARTICLES DESIGNED TO ABSORB BODY FLUIDS**

(30) Priority: 01.04.2014 IT FI20140071
(71) Applicant: Fintex and Partners Italia S.p.A., 51100 Pistoia (IT)
(72) Inventor: Giacometti, Claudio, 55100 Pistoia (IT); Bulleri, Barbara, 51019 Ponte Buggianese (PT) (IT)
(74) Representative: Mannucci, Michele

(57) **Abstract**

A composition for articles designed to absorb body fluids, comprising superabsorbent polymer, characterized by further comprising calcium silicate or a mixture including calcium silicate, an absorbent product comprising said compostion, an absorbent article comprising said product and a method of producing said product or article.

## Description

### Technical Field

The present invention relates to the field of products designed to absorb liquids and, more in particular, body fluids. An object of the invention is a composition for articles designed to absorb body fluids, a product to absorb liquids produced with said composition, as well as an article comprising said product.

A further object of the invention is a method to produce an absorbent product or an absorbent article.

### State of the Art

Polymer-based products with high absorbent capability have been marketed for many years; they are called superabsorbent polymers or, simply, "SAPs", and are particularly suitable for use in diapers, sanitary napkins, incontinence products etcetera.

More specifically, the superabsorbent polymers (SAPs) are materials able to absorb and retain large amount of water or water solutions.

A first type of superabsorbent polymers, no longer widely used, is prepared using hydrophilic polymers with high water-affinity, like modified starch or cellulose, polyvinyl alcohol, polyethylene oxide. Once they have been weakly cross-linked by means of chemical of physical means, they can swell, without being dissolved, in water.

A second type of superabsorbent polymers, that today are most widely used, consists of partially neutralized and weakly cross-linked polymers of the acrylic and methacrylic acid.

Usually, the superabsorbent polymers are marketed in the form of dry powders (humidity content weight usually lower than 10% in proportion to the polymer weight). Once they are into contact with the water, they swell up and form gummy gels that can have a water content greater than 99%.

These products are described, for instance, in the patent documents GB-A-2301350 and EP-A-0891758.

These two documents disclose, for instance, formulations containing SAPs and method for the production of articles containing superabsorbent material. An important aspect of these formulations is the addition of glycerol and water that, after compression, bind together with the superabsorbent powder granules, thus forming a consolidated mass. The superabsorbent material can then be shaped in tablets, or it can be compressed between an upper layer and a lower layer to form a product similar to a sheet.

However, there are practical limits to the amount of glycerol and water that it is possible to add to the SAP.

The patent document EP 1145724B1, for instance, discloses a way to overcome these limits; in particular, it discloses a method to increase the amount of glycerol and water that can be added to the formulation. More in particular, it has been found that, using an adequate amount of silica in the formulation, it is possible to increase the amount of glycerol and water.

These formulations are particularly useful to prepare absorbent mats comprised of an upper layer and a lower layer, both these layers containing cellulose fibers (for example, paper sheets), between which there is a powder composed of SAP, silica, glycerol and water. Through simple compression, these mats are made cohesive and bond together, thus acquiring such a good mechanical consistency to make them particularly suitable for use in diapers, hygienic napkins, incontinence products. it is believed that the cohesion between the materials is due to hydrogen bonds between glycerol and water and the cellulose fibers of the upper and lower layers, the SAP powder and silica.

Silica seems to act as a lubricant, allowing the SAP particles to slide more easily and to be more easily compressed. Moreover, silica allows to incorporate more glycerol and water, thus making the cohesion between the upper and lower cellulose layers more effective.

According to both the patent document GB-A-230135 and EP-A-0891758, a problem arisen in the production of the above described mats is that, after aging, their mechanical resistance decreses. A significant decay of their mechanical features occurs, for instance, after a 6-month aging period at 40°C 75% rH.

Also the documents US2006/178071, US2005/245684 and US2010/268181 disclose similar issues.

### Object and summary of the invention

The scope of the present invention is to provide a composition that is able to absorb a large amount of liquids and is simple to be produced.

Within this scope, a further significant object of the invention is to give high mechanical resistance to the products incorporating the composition, even after aging, preferably as regards hygienic products like diapers, hygienic napkins, incontinence products, absorbent shores, bandages, patches, pockets for the absorption of body fluids and exudates, etcetera.

A further important object of the invention is to provide a flat absorbent article, or absorbent mat, that is suitable to absorb a large amount of body fluids and can be easily produced.

A further significant object of the invention is to provide a flat absorbent article, or absorbent mat, featuring optimal mechanical resistance over time.

This and other objects, that will be better explained below, are achieved through a composition (here below referred to also as "formulation" or "mixture") for articles designed to absorb body fluids, comprising superabsorbent polymer; this composition is characterized by further comprising calcium silicate.

Preferably, in the composition, given 100 parts by weight of said super-absorbent polymer, there are 3 to 20 parts by weight of said calcium silicate or of mixture comprising calcium silicate.

Preferably, given 100 parts by weight of super-absorbent polymer, the amount of calcium silicate or of mixture comprising calcium silicate is at least 3 parts by weight, and more preferably at least 4 parts by weight, and more preferably at least 5 parts by weight, more preferably at least 6 parts by weight, and more preferably at least 7 parts by weight, and in one case at least 8 parts by weight; preferably, given 100 parts by weight of super-absorbent polymer, said product comprising calcium silicate being lower than or equal to 20 parts by weight, more preferably lower than or equal to 15 parts by weight, more preferably lower than or equal to 10 parts by weight and, in one case, lower than or equal to 8 parts by weight.

In preferred embodiments, the mixture comprising calcium silicate furthermore comprises silica.

Preferably, given 100 parts by weight of said super-absorbent polymer, there are 2 to 24 parts by weight of said mixture of calcium silicate and silica.

Preferably, said mixture of silica and calcium silicate provides a ratio of silica to calcium silicate comprised between 0 (only calcium silicate) and 40 (40 parts by weight silica and 1 part by weight calcium silicate), and more preferably between 0.25 (2 parts by weight silica and 8 parts by weight calcium silicate) and 10 (10 parts by weight silica and 1 parts by weight calcium silicate).

Said superabsorbent polymer is preferably sodium polyacrylate cross-linked powder.

Preferably, said super-absorbent polymer powder has a particle distribution comprised between 20 and 900 microns, preferably between 50 and 700 microns, and more preferably between 60 and 600 microns.

Preferably, the mixture of calcium silicate and silica is in powder form and has a particle size lower than 100 microns and preferably lower than 50 microns and more preferably lower than 10 microns.

According to preferred embodiments, the composition comprises polyhydroxy compound.

Preferably, in said composition, given 100 parts by weight of said super-absorbent polymer, there are 3 to 40 parts by weight of said polyhydroxy compound.

Preferably, said polyhydroxy compound is non-volatile, water-soluble or water-dispersible at a temperature equal to or lower than 55°C, liquid at a temperature lower than 31°C.

Preferably, said polyhydroxy compound is one of the following, or a combination of the following: glycerol, 1-2 propanediol, polyethylene glycol 200, polyethylene glycol 400, sorbitol.

The composition preferably comprises water; more preferably, given 100 parts by weight of said super-absorbent polymer, there are in the composition from 0.5 to 7 parts by weight of water.

According to preferred embodiments, the composition further comprises a cohesive mixture able to make the set of said super-absorbent polymer and said product containing calcium silicate consolidable.

This cohesive mixture preferably comprises said polyhydroxy compound. The cohesive mixture preferably comprises water.

According to preferred embodiments, the composition comprises:
SAP 100 parts by weight,
Polyhydroxy compound from 3 to 40 parts by weight,
Water from 0.5 to 7 parts by weight,
Mixture of calcium silicate and silica from 3 to 20 parts by weight.

Preferably, the composition is characterized by being consolidated and by the fact that, after 6-month aging test at 40°C 65% relative humidity, the percentage decrease of its tensile strength is lower than or equal to 35%.

Adequately, the composition is arranged inside hygienic products such as baby diapers, sanitary napkins, incontinence products, absorbent shores, bandages, patches, pockets for the absorption of body fluids and exudates, etcetera.

The composition may also include inert substances like calcium carbonate, bentonites, diatomaceous earth and additives able to give the absorbent material of the invention additional features. The composition may for instance include antibacterial and anti-odor substances, as well as perfume. Examples of these substances are benzalkonium chloride, α-, β-, and γ-cyclodextrins and derivatives thereof, such as metyl cyclodextrins or hydrossipropyl cyclodextrins.

According to a further aspect, the invention relates to an absorbent product comprising a composition according to one or more of the previous configurations.

This absorbent product preferably comprises at least one layer made of a material with said composition.

According to a further aspect, the invention relates to an absorbent article comprising at least one layer with a composition according to one or more of the previous configurations, cohesive and coupled between two sheets in order to take a substantially flat shape.

The absorbent article preferably comprises adhesive between said at least one layer and at least one of said two sheets.

At least one of said two sheets is preferably made of cellulose material; preferably said sheets are tissue paper sheets or air-laid sheets.

The two sheets preferably provide heat-sealable material; the sheets are preferably made of non-woven fabric.

According to a further aspect, the invention relates to a method to produce an absorbent product or article according to one or more of the previous configurations, comprising:
- to mix a super-absorbent polymer with calcium silicate and, preferably, silica, to form a first mixture,
- to add a further cohesive mixture to the first mixture, in order to make this latter consolidable, up to obtain an absorbent product.

The method preferably provides that the absorbent product is distributed on a first sheet and is then covered by a second sheet; this set is then compressed in order to consolidate the absorbent product and to join the sheets to said product, so as to produce a substantially flat absorbent article.

The cohesive mixture is preferably added after the first mixture has been formed. The cohesive formulation preferably includes glycerol and water. As already mentioned, it is possible to use, instead of glycerol, other non-volatile polyhydroxy compounds, water-soluble or water-dispersible at 50°C or less. These compounds shall be liquid at a temperature lower than 30°C.

### Brief description of the drawings

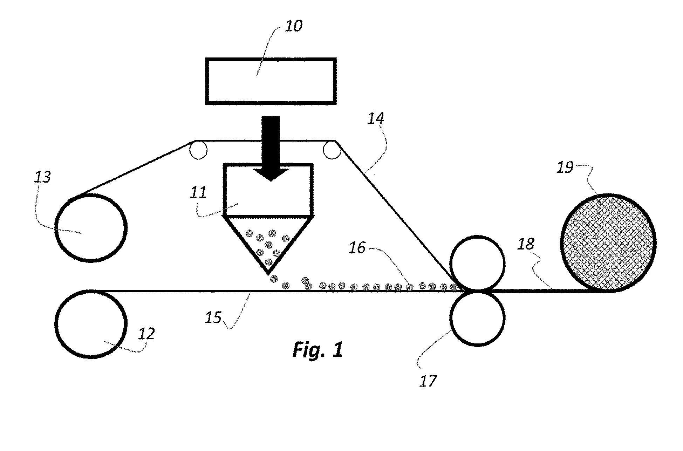

Further characteristics and advantages of the invention will be more apparent from the description of some preferred, although not exclusive, embodiments, illustrated by way of non-limiting example in the attached tables of drawings, wherein: figure 1 is a diagram of a plant to produce a flat absorbent article or mat according to the present invention.

### Detailed description of some embodiments of the invention

Figure 1 shows a schematic drawing of an apparatus suitable to form an absorbent mat containing the composition (here below referred to also as "formulation" or "mixture") of the present invention. In particular, the elements of this formulation can be mixed together in a mixer 10 in order to obtain a homogenous mixture. Then, the mixture containing the superabsorbent polymer (here below referred to also as "SAP") is transferred to a powder dosing machine, that distributes the powder 16 on the sheet of cellulose material 12. This sheet of cellulose material is unwound from a reel 12. A second sheet of cellulose material 14 is unwound from a further reel 13 and is applied on the powder 16 and the sheet of cellulose material 15. In this way a coupled material ("sandwich") is formed, wherein the powder containing superabsorbent polymer is arranged between two sheets of cellulose material. Then, the coupled material is compressed by means of a pair of rollers 17; passing between them, the materials are made permanently cohesive, under the effect of pressure (and temperature, depending upon the specific procedure), in order to form the substantially flat absorbent article or absorbent mat 18, that is then wound to form a reel 19. The apparatus 17 may be also composed of a pair of rollers suitable to emboss the absorbent mat 18. Instead of the reel 19, alternative systems may be used, like spooling or festooning.

### Test method

### Analysis method 1 - Aging test on absorbent mats

For this test a heater is used to keep the temperature constant, with fluctuations of +/-0.5°C and also the relative humidity constant, with fluctuations of +/- 1% rH. The test temperature is 40°C, the relative humidity is 65%. Once the samples have been inserted, the test is performed, lasting for a 6-month period.

### Test method 2 - Measurement of the tensile strength of the absorbent mats

The test is carried out according to the test method EDANA 20.2-89 "Tensile strength".

### Examples

### Example 1

An example of formulation according to the prior art comprises, in parts by weight (pbw):

| | |
|---|---|
| SAP | 100 pbw |
| glycerol | 14 pbw |
| water | 4 pbw |

the superabsorbent polymer used for the test may be the product marketed under the name Hysorb Fem 33M produced by BASF SE 67056 Ludwigshafen Germany.

The formulation is obtained by adding glycerol and water, previously mixed together, to the superabsorbent powder, and then mixing until to obtain a powder.

### Example 2

An example of formulation according to the prior art comprises, in parts by weight (pbw):

| | |
|---|---|
| SAP | 100 pbw |
| silica | 10 pbw |
| glycerol | 14 pbw |
| water | 4 pbw |

the superabsorbent polymer used for the test may be the product marketed under the name Hysorb Fem 33M produced by BASF SE 67056 Ludwigshafen Germany.
The first step is the step of mixing SAP granules and silica together.

In the second step, a ready-mix of glycerol and water is added to the mixture of SAP and silica. During the second step, the formulation is mixed until to achieve a powder consistency.

### Example 3

An example of formulation according to the invention comprises, in parts by weight (pbw):

| | |
|---|---|
| SAP | 100 pbw |
| calcium silicate | 10 pbw |
| glycerol | 14 pbw |
| water | 4 pbw |

the superabsorbent polymer used for the test may be the product marketed under the name Hysorb Fem 33M produced by BASF SE 67056 Ludwigshafen Germany. The calcium silicate used is the product marketed under the name Sipernat 880, produced by Evonik Industries AG Postfach 1345 D-63403 Hanau.

The first step is the step of mixing together superabsorbent granules and calcium silicate.

In the second step, a ready-mixed of glycerol and water is added to the mixture of superabsorbent and calcium silicate. During the second step, the formulation is mixed until to achieve a powder consistency.

### Example 4

An example of formulation according to the invention comprises, in parts by weight (pbw):

| | |
|---|---|
| SAP | 100 pbw |
| calcium silicate | 1 pbw |
| silica | 9 pbw |
| glycerol | 14 pbw |
| water | 4 pbw |

the superabsorbent polymer used for the test may be the product marketed under the name Hysorb Fem 33M produced by BASF SE 67056 Ludwigshafen Germany. The calcium silicate used is the product marketed under the name Sipernat 880, produced by Evonik Industries AG Postfach 1345 D-63403 Hanau.

The first step is the step of mixing SAP granules, calcium silicate and silica together.

In the second step, a ready-mix of glycerol and water is added to the mixture of SAP, calcium silicate and silica. During the second step, the formulation is mixed until to achieve a powder consistency.

### Example 5 - Preparation of the absorbent mats

This example illustrates how to form absorbent mats by using the formulations produced in the previous examples.

With reference to figure 1, the formulations of the previous examples are mixed in a mixer 10 until to have a powder consistency, and are then deposited on a tissue paper sheet moving at a preset speed from a reel of tissue paper 12. A second sheet of tissue paper 14 from a second reel of tissue paper 13 is transported, and both these sheets of tissue paper are arranged so as to have, in the middle thereof, i.e. between them, the powder containing the superabsorbent polymer. The product passes through a pair of rollers 17 and is made permanently cohesive. The product obtained is a flat sheet than can be cut into adequate shapes. Alternatively, the sheet exiting the second cylinder may be wound, at the end of the line, by means of a winding machine to form a reel 19. In other cases, the sheet may be wound at the exit by spooling or festooning.

### Example 6 - Preparation of the absorbent mats for the aging tests

Absorbent mats are produced according to what described in Example 5, these mats comprising the formulations described in the examples 1, 2, 3, and 4. In these formulations, the total grammage of the mat is 500 g/m2, while the component of the pre-mixed formulation containing SAP, prepared according to the formulations of examples 1, 2, 3, and 4, is 420 g/m2. The mats produced through the described process and deriving from the formulations described in the examples 1, 2, 3, and 4, are called M1, M2, M3, and M4 respectively.

### Example 6 - Mat aging test

This example illustrates the measurements of the tensile strength of the absorbent mats produced according to Example 5. The tensile strength test is done on the samples immediately after they have been prepared, as well as after the 6-month aging test has been performed (40°C 65% rH). The results are shown in Table 1. It is clearly apparent that the mats prepared according to the invention maintain good mechanical properties after the aging test.

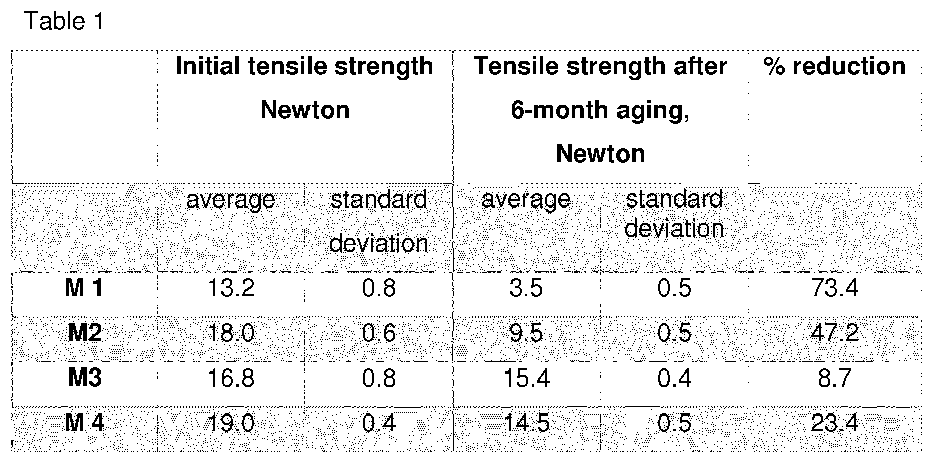

It is clearly apparent that the mats M3 and M4 prepared according to the invention maintain good tensile strength even after an accelerated 6-month aging period at 40°C and 65% relative humidity.

It is understood that what illustrated above purely represents possible non-limiting embodiments of the invention, which may vary in forms and arrangements without departing from the scope of the concept on which the invention is based. Any reference numbers in the appended claims are provided for the sole purpose of facilitating the reading thereof in the light of the description before and the accompanying drawings and do not in any way limit the scope of protection of the present invention.

## Claims

1. A composition for articles designed to absorb body fluids, comprising super-absorbent polymer, **characterized by** further comprising calcium silicate or a mixture including calcium silicate.

2. Composition according to claim 1, wherein, given 100 parts by weight of said super-absorbent polymer, there are 3 to 20 parts by weight of said calcium silicate or of said mixture comprising calcium silicate.

3. Composition according claim 1 or 2, wherein, given 100 parts by weight of super-absorbent polymer, the amount of said calcium silicate or of said mixture comprising calcium silicate is at least 3 parts by weight, and more preferably at least 4 parts by weight, and more preferably at least 5 parts by weight, more preferably at least 6 parts by weight, and more preferably at least 7 parts by weight, and in one case at least 8 parts by weight; preferably, given 100 parts by weight of super-absorbent polymer, said product comprising calcium silicate being lower than or equal to 20 parts by weight, more preferably lower than or equal to 15 parts by weight, more preferably lower than or equal to 10 parts by weight and, in one case, lower than or equal to 8 parts by weight.

4. Composition according to one or more of the previous claims, wherein said composition comprises a mixture comprising calcium silicate and silica.

5. Composition according to claim 4, wherein, given 100 parts by weight of said super-absorbent polymer, there are 2 to 24 parts by weight of said mixture of calcium silicate and silica.

6. Composition according to claim 4 or 5, wherein said mixture provides a ratio of silica to calcium silicate comprised between 0 (only calcium silicate) and 40 (40 parts by weight silica and 1 part by weight calcium silicate), and more preferably between 0.25 (2 parts by weight silica and 8 parts by weight calcium silicate) and 10 (10 parts by weight silica and 1 parts by weight calcium silicate).

7. Composition according to one or more of the previous claims, wherein said super-absorbent polymer is sodium polyacrylate cross-linked powder.

8. Composition according to claim 7, wherein said super-absorbent polymer powder has a particle distribution comprised between 20 and 900 microns, preferably between 50 and 700 microns, and more preferably between 60 and 600 microns.

9. Composition according to claim 5 or to claim 5 and one or more of claims 6 to 8, wherein said mixture of calcium silicate and silica is in powder form and has a particle size lower than 100 microns and preferably lower than 50 microns and more preferably lower than 10 microns.

10. Composition according to one or more of the previous claims comprising polyhydroxy compound; preferably, given 100 parts by weight of said super-absorbent polymer, there are 3 to 40 parts by weight of said polyhydroxy compound; preferably, said polyhydroxy compound is non-volatile, water-soluble or water-dispersible at a temperature equal to or lower than 55°C, liquid at a temperature lower than 31 °C. preferably, said polyhydroxy compound is one of the following: glycerol, 1-2 propanediol, polyethylene glycol 200, polyethylene glycol 400, sorbitol.

11. Composition according to one or more of the previous claims, comprising water; preferably, given 100 parts by weight of said super-absorbent polymer, there are from 0.5 to 7 parts by weight of water.

12. Composition according to claim 11, wherein

13. An absorbent product comprising a composition according to one or more of the previous claims; said absorbent product preferably comprising at least one layer made of a material with said composition.

14. An absorbent article comprising at least one layer with a composition according to one or more of the previous claims, cohesive and coupled between two sheets to assume a plate aspect; said absorbent article preferably comprises adhesive between said at least one layer and at least one of said two sheets; said at least one of said two sheets is preferably made of a cellulose material; preferably said sheets are tissue paper sheets or air-laid sheets.

15. A method for producing a product or an article according to one or more of claims 22 to 27, comprising the following steps:
- to mix a super-absorbent polymer with calcium silicate and, preferably, silica, to form a first mixture,
- to add to the first mixture a further cohesive mixture to make the first mixture consolidable, up to obtain an absorbent product;
preferably to distribute said absorbent product onto a first sheet and to cover it by a second sheet; to compress the set produced in order to consolidate the absorbent product and to join said sheets to said product so as to produce a substantially flat absorbent article.
